# EUROPEAN PATENT SPECIFICATION

(11) **EP 1 330 347 B1**
(45) Date of publication and mention of the grant of the patent: **16.08.2006**
(21) Application number: 01973634.7
(22) Date of filing: 28.09.2001
(51) Int. Cl.: B29C 35/08, B29C 71/00, B29C 71/04, A61F 2/02, A61L 27/14

(54) **SUPERCRITICAL FLUID TREATMENT OF AN IRRADIATED PREFORM OF A PROSTHESIS BEARING MADE OF POLYETHYLENE**
BEHANDLUNG EINES BESTRAHLTEN VORFORMLINGS EINES PROTHETISCHEN LAGERELEMENTES AUS POLYETHYLEN MIT EINEM ÜBERKRITISCHEN FLUID
TRAITEMENT AVEC DU FLUIDE SUPERCRITIQUE D'UNE PREFORME IRRADIE D'UN PALIER PROTHETIQUE DE POLYETHYLENE

(30) Priority: 29.09.2000 US 236983 P
(43) Date of publication of application: 30.07.2003
(73) Proprietor: DEPUY ORTHOPAEDICS, INC., Warsaw, Indiana 46581-0988 (US)
(72) Inventor: RICHARD, Robert, Wrentham, MA 02093 (US)
(74) Representative: Belcher, Simon James
(86) International application number: PCT/US2001/030582
(87) International publication number: WO 2002/026464

(56) References cited:
- EP-A- 0 572 913
- EP-A- 0 722 973
- EP-A- 0 847 765
- EP-A- 0 963 824
- EP-A- 1 072 275
- EP-B- 0 809 564
- WO-A-00/49079
- DE-A- 4 306 645
- US-A- 5 607 518
- US-A- 5 753 182

## Description

### FIELD OF THE INVENTION

The present invention relates to a process for forming orthopaedic implant prosthesis bearings of cross-linked polyethylene, high density polyethylene, high molecular weight polyethylene, high density high molecular weight polyethylene, and ultrahigh molecular weight polyethylene having increased wear resistance and improved mechanical properties. The present invention particularly relates to processes using supercritical fluid-treatment of irradiated polyethylenes.

### BACKGROUND OF THE INVENTION

Ultrahigh molecular weight polyethylene (UHMWPE) has been the material of choice for articulating surface applications for three decades. Such UHMWPE resin is commonly used for implantable prosthesis bearings, such as acetabular bearings, glenoid bearings, tibial bearings, and the like, for use in hip, shoulder, knee, and elbow prostheses. The bearings may be formed from polyethylene by direct compression molding processes or by machining the required bearing shapes from mill shapes such as sheet or bar stock. Molding processes may be performed on unirradiated or irradiated polyethylene. Over time, many improvements have been introduced in regard to the fabrication of such bearings, most notably irradiation of the polyethylene to induce cross-linking. In fact, the improved wear characteristics of the polyethylene have been largely attributed to such cross-linking procedures. Typically, a bar stock or preform, or a molded or machined bearing, is irradiated and subsequently heat treated or heat annealed. The irradiation generates molecular cross-links and free-radicals. Such cross-linking creates a 3-dimensional network in the polymer which renders it more resistant to abrasive wear in multiple directions. In addition, the free radicals formed upon irradiation of UHMWPE can also participate in oxidation reactions, which reduce the molecular weight of the polymer via chain scission, leading to degradation of physical properties, embrittlement, and an increase in wear rate. The free radicals may be very long-lived, often several years, so that oxidation can continue over an extended period of time. Processes that tend to substantially eliminate residual free radicals induced by such irradiation tend to provide polyethylene with improved oxidation resistance. Typical processes for quenching free radicals in polyethylene induced by irradiation involve elimination of the free radicals with heat treatments, as well as prolonged exposure of the irradiated polyethylene to stabilizing gases such as hydrogen. Such process steps may serve to accelerate free radical recombination as well as additional crosslinking reactions in the polymer.

Reference is made to a number of prior art references as follows:
1. U.S. Patent No. 5,728,748, and its counterparts all relating to the same application, "Non-Oxidizing Polymeric Medical Implant," to Sun, et al.
2. U.S. Patent No. 5,879,400, "Melt-Irradiated Ultra High Molecular Weight Polyethylene Prosthetic Devices," to Merrill et al.
3. U.S. Patent No. 6,017,975, "Process for Medical Implant of Cross-Linked Ultrahigh Molecular Weight Polyethylene Having Improved Balance of Wear Properties and Oxidation Resistance," to Saum, et al..
4. U.S. Patent No. 6,228,900, "Crosslinking of Polyethylene for Low Wear Using Radiation and Thermal Treatments," to Shen et al.
5. U.S. Patent No. 6,168,626, "Ultra High Molecular Weight Polyethylene Molded Article for Artificial Joints and Method of Preparing the Same," to Hyon et al.
6. U.S. Patent No. 6,245,276, "Method for Molding a Cross-Linked Preform," to McNulty et al.
7. U.S. Patent No. 6,281,264, "Chemically Crosslinked Ultrahigh Molecular Weight Polyethylene for Artificial Human Joints," to Salovey et al.
8. U.S. Patent No. 5,753,182, "Method for Reducing the Number of Free Radicals Present in Ultrahigh Molecular Weight Polyethylene Orthopedic Components ," to Higgins.

The above references teach the general concepts involved in forming or consolidating polyethylene resin directly into a component or a stock form from which the component is made, gamma or other irradiation of the component or the stock form, subsequent heat treating (including annealing or remelting) of the component or stock form, and conventional methods of quenching of the component or stock form. The above references also teach the general concepts of compression appropriate apparatus used therein.

WO-00/49079 discloses a method for treating an ultrahigh molecular weight polyethylene for use in a joint prosthesis which involves exposure to a solution of an antioxidant in a supercritical fluid, prior to an irradiation step.

### SUMMARY OF THE INVENTION

The present invention provides polyethylene bearings with improved mechanical properties, improved oxidation resistance, and increased wear resistance. The polyethylenes prepared by the processes of the present invention can also reduce the amount of wear debris generated from such bearings. Typically, the polyethylene may be ultrahigh molecular weight polyethylene (UHMWPE), although it will be appreciated that the processes of the present invention may be used with various types of polyethylene. The term "polyethylene," as defined herein, includes polyethylene, high density polyethylene, high molecular weight polyethylene, high density high molecular weight polyethylene, ultrahigh molecular weight polyethylene, or any other type of polyethylene utilized in the construction of a prosthetic implant.

The present invention is directed to a process for preparing polyethylene suitable for applications requiring high resistance to abrasive wear. In particular, the present invention is directed to a process for preparing polyethylene suitable for articular surfaces and orthopaedic bearings by treating an irradiated polyethylene with a supercritical fluid (SCF). What is meant herein by the term "bearing" is an orthopaedic implant prosthetic bearing of any type, condition, shape, or configuration. The SCF treatment is performed at appropriate temperatures and pressures consistent with forming supercritical fluids, as described below. Optionally, the SCF may be mixed with other permanent gases, such as hydrogen, nitrogen, and the like during the free radical quenching process.

In some embodiments, preforms for the fabrication of prosthesis bearings may be made from consolidated polyethylene stock which has been irradiated. In other embodiments, the polyethylene stock may be pre-annealed or pressure crystallized, or a combination thereof, to further enhance its mechanical properties. In still other embodiments, instead of a preform, a formed bearing is cross-linked by irradiation and SCF-quenched as described below.

The present invention further pertains to improved cross-linked polyethylene that can be made by the processes described herein. In particular, ultrahigh molecular weight polyethylene (UHI1ZWPE) prepared by the processes of the present invention illustratively exhibits high yield strength, high ultimate tensile strength, and high impact resistance. UHMWPE prepared by the processes of the present invention can exhibit a swell ratio of about 5 or less and a percent elongation to break of about 250% or greater, or preferably a percent elongation to break of about 300% or greater. It is appreciated that a percent elongation to break greater than about 400% may be achieved under certain conditions. This UHMWPE also has a low residual free radical population, thus possessing oxidation resistance comparable to UHMWPE prior to irradiation. Bearings fabricated from UHMWPE prepared by the processes described herein can exhibit increased wear resistance and improved mechanical properties.

Additional features of the present invention will become apparent to those skilled in the art upon consideration of the following detailed description of invention exemplifying the best mode of carrying out the invention as presently perceived.

### BRIEF DESCRIPTION OF THE DRAWINGS

Fig. 1 is a schematic view of an implantable prosthetic bearing that may be produced by processes described herein;
Fig. 2 is a perspective view of an implantable glenoid bearing prosthesis that may be produced by processes described herein;
Fig. 3 is a perspective view of an implantable acetabular bearing prosthesis that may be produced by processes described herein;
Fig. 4 is a perspective view of an implantable tibial bearing prosthesis that may be produced by processes described herein; and
Fig. 5 is a pressure-temperature phase diagram which illustrates the critical point and the associated supercritical fluid region.

### DETAILED DESCRIPTION OF THE INVENTION

A typical prosthetic bearing design includes an articulating or bearing surface on which either a natural bone structure or a prosthetic component articulates. In addition, a typical prosthetic bearing design also includes an engaging surface which may include locking features in the form of mechanisms such as pins, tabs, tapered posts, or the like for locking or otherwise securing the bearing to either another component associated with a prosthetic assembly (e.g., a metal shell or tray) or to the bone itself.

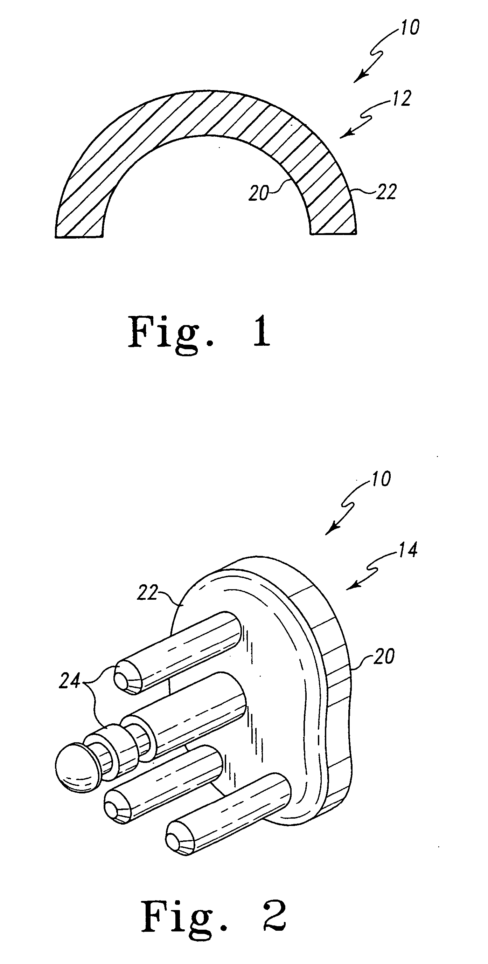

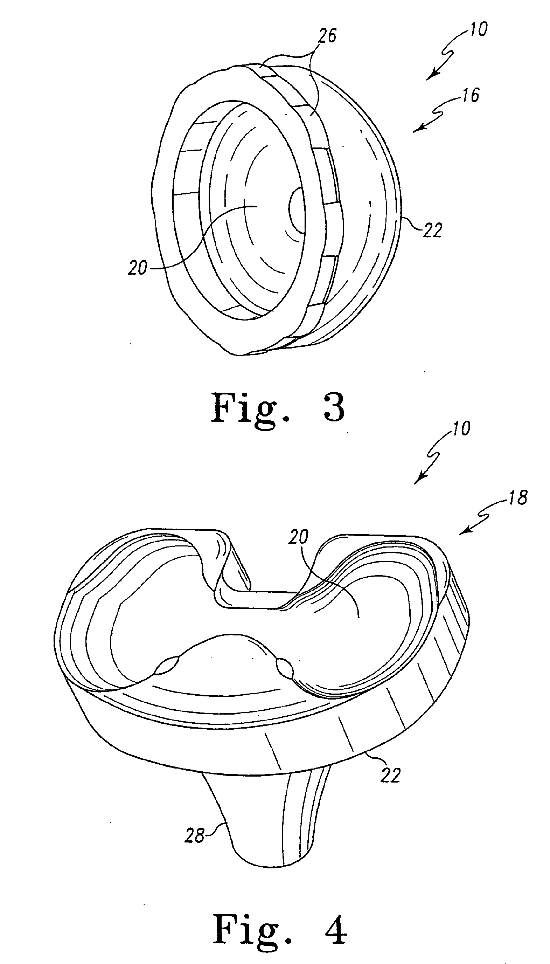

Referring now to Figs. 1-4, there is shown an implantable prosthetic bearing 10. The bearing 10 is shown schematically as a bearing 12 in Fig. 1, whereas specific exemplary embodiments of the prosthetic bearing 10, such as a glenoid bearing 14 for implantation into a glenoid of a patient (not shown), an acetabular bearing 16 for implantation into an acetabulum of a patient (not shown), and a tibial bearing 18 for implantation into a tibia of a patient (not shown) are shown in Figs. 2-4, respectively. Each of the embodiments of the prosthetic bearing 10 includes an articulating or bearing surface 20 on which a natural or prosthetic component bears. For example, in the case of the glenoid bearing 14, a natural or prosthetic humeral head (not shown) bears on the articulating surface 20. Similarly, in the case of a acetabular bearing 16, a natural or prosthetic femoral head (not shown) bears on the articulating surface 20. Moreover, in the case of the tibial bearing 18, a pair of natural or prosthetic femoral condyles (not shown) bear on the articulating surface 20.

Each of the prosthetic bearings 10 also includes an engaging surface 22 which may have a number of features defined therein for engaging either another prosthetic component or the bone into which the bearing 10 is to be implanted: For example, in the case of the glenoid bearing 14, a number of pins or pegs 24 may be defined in the engaging surface 22 thereof. The pegs 24 are received into a number of corresponding holes (not shown) formed in the glenoid surface of the patient. The pins 24 are typically held in place with the use of bone cement. Moreover, if the glenoid bearing 14 is utilized in conjunction with an implanted metal shell, the engaging surface 22 of the bearing 14 may be configured with a tapered post (not shown) or the like for securing the glenoid bearing 14 to the shell.

In the case of the acetabular bearing 16, a number of keying tabs 26 are defined in the engaging surface 22 along the outer annular surface thereof. The keying tabs 26 are received into a number of corresponding keying slots (not shown) defined in an implanted metal acetabular shell (not shown) in order to prevent rotation of the acetabular bearing 16 relative to the implanted shell. In the case of fixation of the acetabular bearing 16 directly to the acetabulum of the patient (i.e., without the use of a metal shell), the engaging surface 22 of the bearing 16 may alternatively be configured with a number of posts or pegs (not shown) which are received into a number of corresponding holes formed in the patient's acetabulum. In such a case, the posts or pegs are typically held in place with the use of bone cement. Moreover, it should be appreciated that the acetabular bearing 16 may be cemented to the patient's acetabulum without the use of posts or pegs on the engaging surface 22 thereof.

In the case of the tibial bearing 18, a tapered post 28 is defined in the engaging surface 22 thereof. The tapered post 28 is received into a corresponding tapered bore (not shown) defined in an implanted tibial tray (not shown) of a knee prosthesis (not shown). It should be appreciated that the engaging surface 22 of the tibial bearing 18 may also be configured with features to allow the tibial bearing 18 to be secured directly to the tibia without the use of an implanted tray (e.g., by use of bone cement). Moreover, it is appreciated that a tibial bearing for use with a tibial tray may also be designed without the use of the post 28.

The present invention pertains to fabrication of such an orthopaedic implant prosthetic bearing 10 from irradiated polyethylene treated with a SCF. Alternatively, a formed bearing may be irradiated and treated with a SCF. In either case, the preform or formed bearing may be fabricated from an olefinic resin, typically a polyethylene resin, such as an ultrahigh molecular weight polyethylene (UHIVIWPE) resin. It is further appreciated that other polyethylenes such as high molecular weight polyethylene, high density polyethylene, high molecular weight high density polyethylene, and the like may be fabricated into bearings using the processes described herein. The term "preform" as used herein refers to an article that has been consolidated, such as by ram extrusion or compression molding of polyethylene resin particles into rods, sheets, blocks, slabs, or the like. The term "preform" also includes a preform "puck" which may be prepared by intermediate machining of a commercially available preform. Such preforms may be obtained or machined from commercially available UHMWPE, for example GUR 1050 HP ram extruded UHMWPE rods from PolyHi Solidur (Fort Wayne, Indiana). The starting preform may be pressure recrystallized as described in U.S. Patent 5,478,906 and in U.S. Patent 6,017,975. The starting preform may be optionally annealed, as described in U.S. Patent 6,017,975, prior to irradiation. This pre-annealing step may be conducted in a substantially oxygen-free atmosphere. It is appreciated that the preform of the present invention may be formed from a wide variety of crude or processed plastic resins suitable for use in orthopaedics, that can be converted by manufacture into a finished bearing. It is further appreciated that the current invention contemplates cross-linking of the polyethylene prior to intermediate machining of a commercial stock into a preform puck.

An exemplary embodiment of the present invention includes a process that includes the steps of irradiating a polyethylene preform to form free radicals and cross-link the polyethylene, and treating the irradiated preform with a supercritical fluid (SCF) at temperatures and pressures consistent with such SCF's to substantially eliminate free radicals remaining from the irradiation step. Treatment of the polyethylene with SCF's may effect further cross-linking in the polyethylene. Thereafter a bearing may be formed from the irradiated and SCF-quenched preform. Alternatively, an existing formed polyethylene bearing is irradiated to cross-link the polyethylene and the residual free radicals are subsequently quenched by treatment with a SCF.

Preferred temperatures for processing are such that deformation of the formed bearing does not occur, and preferred pressures are such that they are uniform and thus do not deform the formed bearing. However, in the case of the quenching of a preform or a bearing that requires an additional amount of processing or manipulation, such as machining, temperatures above the melting temperature of the polyethylene or pressures that are not substantially uniform may be utilized in the processes described herein.

As alluded to above, the preform or formed bearing is generally irradiated, preferably with gamma radiation; however electron beam or x-ray radiation may also be used. The preform or formed bearing is preferably irradiated in the solid state with gamma radiation at a dose from about 0.5 Mrad to about 50 Mrad using methods known in the art. Alternatively, the preform or formed bearing may be irradiated at a dose from about 1.5 Mrad to about 15 Mrad, or from about 5 Mrad to about 10 Mrad. It will be appreciated that doses of radiation lower than about 0.5 Mrad or higher than about 50 Mrad may be used to prepare certain polyethylenes and in variations of the process. The irradiation process is generally performed at room temperature, however higher temperatures may be used. The irradiation process may be optionally performed under vacuum or in an inert or substantially oxygen-free atmosphere by placing the preform in a bag, which includes materials such as aluminum foil, polyethylene, and the like, suitable for such irradiation processes. The bag may be optionally evacuated and the atmosphere substantially replaced with an inert gas such as nitrogen, argon, and the like. It will be appreciated, however, that acceptable results may be achieved for certain bearing configurations when the irradiation process is carried out under atmospheric conditions, i.e., with some oxygen present. Since the processes described herein allow for radiation-cross-linking of the polyethylene preform prior to forming the bearing, low levels of surface oxidation can be tolerated as the oxidized surface can be removed during subsequent machining of the bearing.

It is appreciated that the preform may be "pre-irradiated" prior to use thereof. In particular, it may be desirable for a manufacturer of prosthetic bearings to purchase material (e.g. polyethylene) which has been irradiated or otherwise cross-linked by a commercial supplier or other manufacturer of the material. Such "out-sourcing" of the irradiation process is contemplated for use in the processes described herein.

In any case, after the polyethylene has been irradiated, it is treated with a SCF, at temperatures and pressures consistent with forming supercritical fluids. The polyethylene is treated with the SCF for a time that is sufficient to recombine substantially all of the free radicals which remain in the material from the irradiation cross-linking process. Such treatment often results in further cross-linking of the polyethylene and its stabilization with regard to oxidation. Supercritical fluids are known to affect the physical dynamics of polymers; in particular, they may effect swelling of polymers. The dissolution and subsequent fractionation of high density polyethylene by supercritical and near-critical propane is disclosed by Watkins *et al.,* in *The Journal of Supercritical Fluids, 1991, 4,* 24-31.

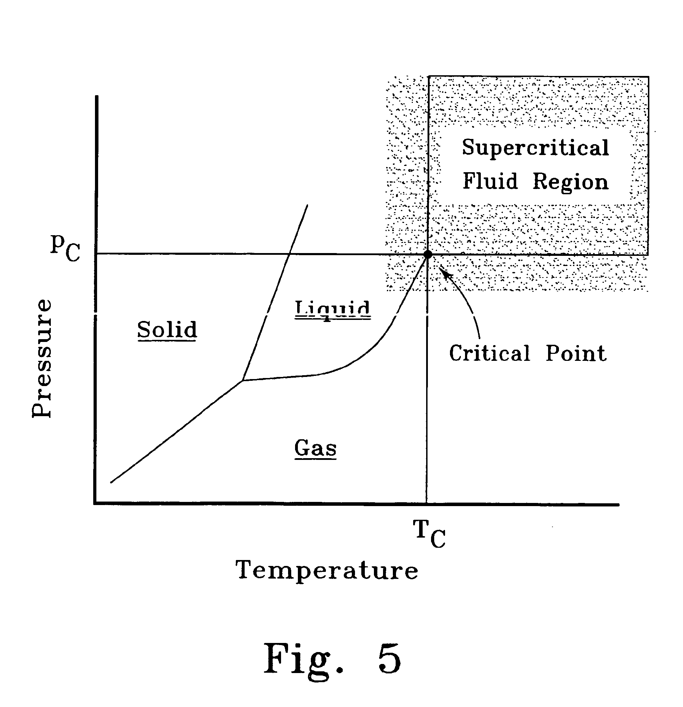

A supercritical fluid is defined herein as a substance where, at a particular temperature, defined as the critical temperature (T_{c}), and at a particular pressure, defined as the critical pressure (p_{c}), the molar volume of the liquid and gaseous phases of the substance are identical. Thus, the distinction between liquid and gaseous phase has been lost and the resulting substance exists as a homogenous "fluid" phase which possesses properties intermediate between the gaseous and the liquid phases. With reference to Fig. 5, the point on the pressure-temperature phase diagram defined by temperature T_{c} and pressure p_{c} is the critical point. Above T_{c}, the substance can no longer be condensed at any pressure into a liquid phase. The "supercritical region" is defined herein to include pressure and temperature ranges dictated by the area present on the Temperature - Pressure phase diagram bound by extrapolation above and to the right of the critical point, as shown in the solid-outlined box of Figure 5. In addition, the gaseous region below the critical pressure extrapolation along with the liquid region to the left of the critical temperature extrapolation may also, under certain conditions, possess supercritical fluid-like characteristics; As a result, these regions, which are commonly utilized to describe "near-critical fluids" and "subcritical fluids", are therefore contemplated for inclusion into the term "supercritical fluid" as used herein. For example, the region of temperatures and pressures designated in the shaded area of Fig., 5 indicates a region which provides the desirable characteristics of a SCF. Some examples of substances that are useful as supercritical fluids are listed in Table I.

**Table I. Critical points for selected substances useful as supercritical fluids.**

| Substance | Critical temperature (T_{c}, °C) | Critical pressure (p_{c}, MPa) | Critical pressure (p_{c}, psi) |
|---|---|---|---|
| water | 374 | 22.13 | 3210 |
| ammonia | 133 | 11.38 | 1650 |
| Freon 22® | 112 | 4.12 | 598 |
| ethane | 32 | 4.91 | 712 |
| propane | 97 | 4.30 | 624 |
| nitrous oxide | 37 | 7.17 | 1040 |
| carbon dioxide | 31 | 7.38 | 1070 |
| fluoroform | 26 | 4.90 | 711 |
| xenon | 17 | 5.80 | 841 |

The irradiated polyethylene may also be treated with a SCF mixed with other permanent gases, such as hydrogen, nitrogen, and the like, during the free radical quenching process. The irradiated polyethylene is treated at temperatures and pressures consistent with forming supercritical fluids for such mixtures. The irradiated polyethylene is treated for a time that is sufficient to recombine substantially all of the free radicals which remain in the material from the irradiation cross-linking process, thus further cross-linking the material and stabilizing the polyethylene with regard to oxidation. It is appreciated that the addition of permanent gases or stabilizing gases, such as those described herein, to the SCF may affect the quenching process by having an impact on polymer swelling. In addition, it is appreciated that the addition of permanent gases or stabilizing gases to the SCF may affect the quenching process by effectively lowering the critical temperature or critical pressure relative to the temperature and pressure needed to generate the pure SCF. The component of the stabilizing gas, such as hydrogen gas, may be present in from about 0.1% to about 4% by weight, or from about 0.1% to about 1.9% by weight.

Thermal distortion of the UHMWPE formed bearings during SCF treatment likely does not occur at the modest temperatures required for formation of many SCF's. Moreover, given the homogeneous nature of SCF's, deformation of the formed bearing is equally unlikely to occur due to the absence of non-uniform forces exerted by the pressures used in the present invention.

The irradiated polyethylene preferably is treated with a SCF selected from a group consisting of hydrocarbons, fluorocarbons, chlorofluorocarbons, carbon dioxide, nitrous oxide, ammonia, water, and xenon. Preferably, the SCF is selected from a group consisting of hydrocarbons, fluorocarbons, and chlorofluorocarbons. More preferably, the SCF is a hydrocarbon. In an embodiment the supercritical fluid includes hydrogen within the range from 0.1 to 4% by weight. In another embodiment, the process includes the step of heating the preform, prior to exposure to the supercritical fluid, preferably to a temperature from 250° to 350°C, for at least 0.5h. The polyethylene preform or formed bearing is treated at a temperature near the T_{c} for the given supercritical fluid, preferably at a temperature of about 50°C to about 200°C. The polyethylene preform or formed bearing is treated at a pressure near the P_{c} for the given supercritical, preferably about 3.45 MPa to about 34.5 MPa (about 500 psi to about 5000 psi) for about 4 hours or less, preferably for about 2 hours or less. It is appreciated that temperatures below 50°C or above 200°C may be desirable for some supercritical fluids in variations of the present process.

In an embodiment, the irradiation step involves exposing the polyethylene to a dose of gamma radiation within the range from 0.5 Mrad to 50 Mrad.

An exemplary process includes the irradiation of the preform or formed bearing with a dose of radiation as described above, illustratively from about 1,5 Mrad to about 15 Mrad, followed by treatment with a supercritical hydrocarbon at about 6.89 MPa to about 20.68 MPa (about 1000 to about 3000 psi), optionally containing hydrogen gas, at about 80°C to about 100°C for a period of about 2 hours or less. The temperature and hold time that is sufficient to eliminate substantially all of the residual free radicals present in the UHMWPE may be determined by measuring the free radical population present in the samples using the electron paramagnetic resonance (EPR). The temperature and hold time are chosen such that the free radical populations measured by EPR as described below, are decreased by about 90%, preferably decreased by about 95%, or by about 97%, from that population measured by EPR after irradiation and before quenching. Such SCF-quenching treatment after irradiation results in improved molecular mobility, allowing increased cross-linking, and thus, can reduce the oxidation potential of the polyethylene. In an embodiment, the step of exposure to the supercritical fluid is performed at a temperature within the range of from 50°C to 250°C, preferably from 80 to 130°C, for a time of not more than 4h. When conventional heat treatment alone is carried out at comparable temperatures to those used in processes described herein, elimination of free radicals is less complete resulting in higher oxidation potential and increased wear rates.

After SCF treatment, the quenched and cross-linked polyethylene may be cooled, optionally in a substantially oxygen-free atmosphere or vacuum. In an embodiment, the process includes the step of cooling the polyethylene preform to a temperature below the melting temperature of the polyethylene preform, where the cooling step is performed after the heating step, an includes cooling the polyethylene perform at a cooling rate of 40°C per hour or less. The cross-linked polyethylene may be cooled to a temperature less than about 50°C, preferably to about room temperature, prior to exposing the polyethylene to air. In the case of a polyethylene preform, after cooling, the preform is formed into a bearing using processes known in the art such as machining or molding. The cross-linked UHMWPB is especially useful as a bearing surface, for example in prosthetic hip joint cups and as other prosthetic shapes for replacement of other joints of the human body, including knees, shoulders, fingers, spine, and elbows. The finished bearing can be packaged and sterilized.

A more complete understanding of the present invention can be obtained by referring to the following illustrative examples.

### EXAMPLES

### Example I. Hydrocarbon SCF swelling ofUHMWPE

Test samples consisting of small rods (36 mm long and 4.6 mm in diameter) of ram extruded GUR 1020 UHMWPE from Perplas Medical, Bacup England, were exposed to supercritical propane or supercritical ethane at various temperatures and pressures in a small volume pressure vessel equipped with a view port. The samples were suspended in the pressure vessel (Jerguson Gage, Newport Scientific) near the view port and the dimensional changes (length and diameter) of each sample occurring during contact with the SCF were measured through the view port using calipers.

The data in Table II illustrate the effect of contacting UHMWPE with supercritical ethane or propane at various temperatures and pressures for various lengths of time.

**Table II. Percent change in volume of UHMWPE after exposure to supercritical hydrocarbon.**

| Hydrocarbon | Temperature (°C) | Pressure (MPa) | Pressure (psi) | Treatment time (min) | Volume Change (%) |
|---|---|---|---|---|---|
| ethane | 100 | 9.65 | 1400 | 30 | 3 |
| ethane | 60-64 | 17.24 | 2500 | 45 | 9 |
| propane | 95 | 17.24 | 2500 | 45 | 11 |
| propane | 90-92 | 16.55 | 2400 | 30 | 11 |
| propane | 100-103 | 16.55 | 2400 | 60 | 16 |
| propane | 100-103 | 18.62 | 2700 | 30 | 19 |
| propane | 100 | 19.31 | 2800 | 10 | minimal |

### Example 2. Hydrocarbon SCF treatment of irradiated UHMWPE.

Test samples consisting of small rods (36 mm long and 4.6 mm in diameter) of ram extruded GUR 1020 UHMWPE from Perplas Medical, Bacup England, were vacuum packaged in heat sealed aluminum foil pouches. The samples were gamma irradiated at a target dose of 5 Mrad at Isomedix, of Whippany, New Jersey. Following irradiation, the samples were removed from the vacuum packages and exposed to supercritical propane or supercritical ethane at various temperatures and pressures in a small volume pressure vessel equipped with a view port. The samples were suspended in the pre-heated pressure vessel (Jerguson Gage, Newport Scientific) and the appropriate gas was introduced until the desired pressure was attained. After treatment with the SCF, each sample was analyzed with a Bruker EMX EPR spectrometer. The samples were inserted into 5 mm quartz EPR tubes for measurement and the assessment of relative free radical concentration was made by integrated intensity.

The data in Table III illustrate the effect of contacting irradiated UHMWPE with supercritical hydrocarbon at various temperatures and pressure for various length of time. A rapid decrease in the EPR signal was observed along with a measured volume increase of 10-12 % while under SCF conditions. After 90 minutes the relative free radical concentration was reduced by at least 90%. In contrast, irradiated UHMWPE held at 80°C for 90 minutes in an air oven showed only a 69% decrease in the EPR signal.

**Table III. Reduction in free radical population present in irradiated UHMWPE after exposure to supercritical hydrocarbon.**

| Hydrocarbon | Temperature (°C) | Pressure (MPa) | Pressure (psi) | Treatment time (min) | Reduction in free radical population (%) |
|---|---|---|---|---|---|
| ethane | 80 | 10.34 | 1500 | 13 S | 94 |
| ethane | 80 | 20.68 | 3000 | 135 | 95 |
| propane | 80 | 10.34 | 1500 | 30 | 90 |
| propane | 80 | 10.34 | 1500 | 60 | 90 |
| propane | 80 | 10.34 | 1500 | 90 | 92 |
| propane | 80 | 10.34 | 1500 | 120 | 91 |
| propane | 80 | 20.68 | 3000 | 10 | 94 |
| propane | 80 | 20.68 | 3000 | 30 | 90 |
| propane | 80 | 20.68 | 3000 | 60 | 90 |
| propane | 80 | 20.68 | 3000 | 90 | 90 |
| propane | 80 | 20.68 | 3000 | 120 | 91 |

### Example 3. Treatment of irradiated UHMWPE with supercritical hydrocarbon and hydrogen mixture.

The test samples were irradiated as described in Example 2, but following irradiation the test samples were removed from the vacuum packages and exposed to supercritical propane or supercritical ethane containing various weight percentages of hydrogen gas. The samples were suspended in the pre-heated pressure vessel used in Example 2, hydrogen gas was introduced, and the appropriate gas was introduced until the desired pressure was attained. The data in Table IV indicate an improvement in the free radical decay in the presence of hydrogen. It is appreciated that the slightly higher temperature used may also have contributed to the faster free radical decay rate.

**Table IV. Reduction in free radical population present in irradiated UHMWPE after exposure to supercritical hydrocarbon and hydrogen mixtures at 20.68 MPa (3000 psi).**

| Hydrocarbon | Hydrogen (Weight %) | Temperature (°C) | Treatment Time (min.) | Reduction in Free Radical Population (%) |
|---|---|---|---|---|
| -* | 100 | 60 | 70 | 78 |
| - * * | 100 | 100 | 40 | 95 |
| ethane | 0.04 | 80 | 60 | 94 |
| ethane | 0.21 | 80 | 70 | 93 |
| ethane | 1.0 | 80 | 90 | 93 |
| ethane | 2.0 | 80 | 90 | 94 |
| ethane | 4.1 | 100 | 30 | 97 |
| ethane | 4.1 | 100 | 60 | 97 |
| propane | 1.9 | 100 | 15 | 97 |
| propane | 1.9 | 100 | 30 | 98 |
| propane | 1.9 | 100 | 60. | 98 |
| propane | 1.9 | 100 | 90 | 98 |
| propane | 1.9 | 100 | 120 | 99 |

| | | | | |
|---|---|---|---|---|
| * Pure hydrogen gas at 0.21 MPa (30 psi). | | | | |
| ** Pure hydrogen gas at 12.76 MPa (1850 psi). | | | | |

### Example 4. Comparison of treating irradiated UHMWPE with heat or a supercritical propane and hydrogen mixture.

One set of test samples was again treated as described in Example 3 in supercritical propane containing 1.9 weight % hydrogen at 100°C and 20.68 MPa (3000 psi). A second set of test samples were irradiated as described in Example 2, but following. irradiation the test samples were treated with heat alone at 100°C in the vacuum package. It is appreciated that hydrogen may be present in such vacuum packages as a consequence of the irradiation step. The data in Table V illustrate a more rapid decay of free radical populations in SCF treated samples compared to conventional heat treatments.

**Table V. Reduction in free radical population present in irradiated UHMWPE after exposure to a supercritical hydrocarbon and hydrogen mixture (1.9 weight %) at 100°C and 3000 psi compared to heat treatment alone at 100°C in a vacuum package.**

| Treatment Time (min) | SC-Propane/Hydrogen (% Reduction) | Oven Heated (% Reduction) |
|---|---|---|
| 15 | 97 | 64 |
| 30 | 98 | 73 |
| 60 | 98 | 80 |
| 90 | 98 | 84 |
| 120 | 99 | 86 |

Although it has been described herein to cross-link materials via irradiation, a process which has numerous advantages in regard to the present invention, it should be appreciated that certain of such advantages may be achieved by cross-linking the materials by any other suitable technique.

Furthermore, while the processes described herein are presented in the context of quenching free radicals generated during a cross-linking process, it should be appreciated that such a free radical quenching process may be generally applicable to reducing free radical populations which are present whether or not the polyethylene has been cross-linked.

## Claims

1. A process for preparing an orthopaedic bearing, which comprises:
irradiating a polyethylene preform, and
exposing the irradiated preform to a supercritical fluid in order to quench the free radical population that is present in the polyethylene.

2. A process as claimed in claim 1, in which the irradiation step involves exposing the polyethylene to a dose of gamma radiation within the range from 0.5 Mrad to 50 Mrad.

3. A process as claimed in claim 1, in which the supercritical is fluid selected from the group consisting of hydrocarbons, fluorocarbons, chlorofluorocarbons, carbon dioxide, nitrous oxide, ammonia, water, and xenon.

4. A process as claimed in claim 3, in which the supercritical fluid is selected from the group consisting of ethane and propane.

5. A process as claimed in claim 1, in which the supercritical fluid which is used in the quenching step includes a stabilising gas, preferably hydrogen.

6. A process as claimed in claim 5, in which the supercritical fluid includes hydrogen within the range from 0.1 to 4% by weight.

7. A process as claimed in claim 1, in which the preform is formed from an ultrahigh molecular weight polyethylene.

8. A process as claimed in claim 1, which includes the step of forming the bearing from the preform before the step of exposure to the supercritical fluid.

9. A process as claimed in claim 1, which includes the step of heating the preform, prior to the step of exposure to the supercritical fluid, preferably to a temperature from 250° to 350°C, for at least 0.5 h.

10. A process as claimed in claim 9, in which the heating step is performed in a substantially oxygen-free atmosphere.

11. A process as claimed in claim 10, which includes the step of cooling the polyethylene preform to a temperature below the melting temperature of the polyethylene preform, where the cooling step is performed after the heating step, and includes cooling the polyethylene preform at a cooling rate of 40°C per hour or less.

12. A process as claimed in claim 1, in which the step of exposure to the supercritical fluid is performed at a temperature within the range from 50° to 250°C , preferably from 80° to 130°C, for a time of not more than 4 h.

13. A process as claimed in claim 12, in which the step of exposure to the supercritical fluid is performed at a pressure within the range from 3.45 to 27.6 MPa (500 to 4000 psi), preferably from 6.89 to 20.68 MPa (1000 to 3000 psi).

## Patentansprüche

1. Verfahren zum Herstellen eines orthopädischen Lagerelements, welches umfasst:
Bestrahlen eines Polyethylenvorformlings und
Exponieren des bestrahlten Vorformlings gegenüber einem überkritischen Fluid, um die Population freier Radikale zu quenchen, die in dem Polyethylen vorliegt.

2. Verfahren nach Anspruch 1, bei welchen der Bestrahlungsschritt ein Exponieren des Polyethylens gegenüber einer Dosis an Gammastrahlung innerhalb des Bereichs von 0,5 Mrad bis 50 Mrad einschließt.

3. Verfahren nach Anspruch 1, bei welchem das überkritische Fluid ausgewählt wird aus der Gruppe bestehend aus Kohlenwasserstoffen, Fluorkohlenstoffen, Chlorfluorkohlenstoffen, Kohlendioxid, Distickstoffoxid, Ammoniak, Wasser und Xenon.

4. Verfahren nach Anspruch 3, bei welchem das überkritische Fluid ausgewählt wird aus der Gruppe bestehend aus Ethan von Propan.

5. Verfahren nach Anspruch 1, bei welchem das überkritische Fluid, das in dem Quenchschritt verwendet wird, ein Stabilisierungsgas, bevorzugt Wasserstoff, einschließt.

6. Verfahren nach Anspruch 5, bei welchem das überkritische Fluid Wasserstoff innerhalb des Bereichs von 0,1 bis 4 Gewichtsprozent einschließt.

7. Verfahren nach Anspruch 1, bei welchem der Vorformling aus einem Polyethylen mit ultrahohem Molekulargewicht gebildet wird.

8. Verfahren nach Anspruch 1, welches den Schritt eines Bildens des Lagerelements aus dem Vorformling vor dem Schritt der Exposition gegenüber dem überkritischen Fluid einschließt.

9. Verfahren nach Anspruch 1, welches den Schritt eines Erwärmens des Vorformlings, vor dem Schritt der Exposition gegenüber dem überkritischen Fluid, bevorzugt bei einer Temperatur von 250° bis 350°C, für wenigstens 0,5 h einschließt

10. Verfahren nach Anspruch 9, bei welchem der Erwärmungsschritt in einer im wesentlichen sauerstofffreien Atmosphäre durchgeführt wird.

11. Verfahren nach Anspruch 10, welches den Schritt eines Kühlens des Polyethylenvorformlings auf eine Temperatur unter der Schmelztemperatur des Polyethylenvorformlings einschließt, wo der Kühlschritt nach dem Erwärmungsschritt durchgeführt wird und ein Kühlen des Polyethylenvorformlings mit einer Külilgeschwindigkeit von 40°C pro Stunde oder weniger einschließt.

12. Verfahren nach Anspruch 1, bei welchem der Schritt des Exponierens gegenüber dem überkritischen Fluid bei einer Temperatur innerhalb des Bereichs von 50° bis 250°C, bevorzugt von 80° bis 130°C, für eine Zeit von nicht mehr als 4 Stunden durchgeführt wird.

13. Verfahren nach Anspruch 12, bei welchem der Schritt des Exponierens gegenüber dem überkritischen Fluid bei einem Druck innerhalb des Bereichs von 3,45 bis 27,6 MPa (500 bis 4000 psi), bevorzugt von 6,89 bis 20,68 MPa (1000 bis 3000 psi) durchgeführt wird,

## Revendications

1. Procédé de préparation d'un palier prothétique, qui comprend :
l'irradiation d'une préforme en polyéthylène, et
l'exposition de la préforme irradiée un fluide supercritique afin de désactiver la population de radicaux libres qui est présente dans le polyéthylène.

2. Procédé selon la revendication 1, dans lequel l'étape d'irradiation implique l'exposition du polyéthylène à une dose de rayonnement gamma situé dans la plage de 0,5 Mrad 50 Mrad.

3. Procédé selon la revendication 1, dans lequel le fluide supercritique est un fluide choisi dans le groupe constitué par des hydrocarbures, des fluorocarbones, des chlorofluorocarbones, le dioxyde de carbone, l'oxyde nitreux, l'ammoniac, l'eau et le xénon.

4. Procédé selon la revendication 3, dans lequel le fluide supercritique est choisi dans le groupe constitué par l'éthane et le propane.

5. Procédé selon la revendication 1, dans lequel le fluide supercritique qui est utilisé dans l'étape de désactivation comprend un gaz de stabilisation, de préférence l'hydrogène.

6. Procédé selon la revendication 5, dans lequel le fluide supercritique comprend de l'hydrogène situé dans la plage de 0,1 à 4 % en poids,

7. Procédé selon la revendication 1, dans lequel la préforme est formée à partir d'un polyéthylène de poids moléculaire très élevé.

8. Procédé selon la revendication 1, qui comprend l'étape de formation du palier à partir de la préforme avant l'étape d'exposition au fluide supercritique.

9. Procédé selon la revendication 1, qui comprend l'étape de chauffage de la préforme, avant l'étape d'exposition au fluide supercritique, de préférence à une température de 250° à 350°C, pendant au moins 0,5 heure.

10. Procédé selon la revendication 9, dans lequel l'étape de chauffage est réalisée sous une atmosphère essentiellement exempte d'oxygène.

11. Procédé selon la revendication 10, qui comprend l'étape de refroidissement de la préforme en polyéthylène jusqu'à une température inférieure à la température de fusion de la préforme en polyéthylène, où l'étape de refroidissement est réalisée après l'étape de chauffage, et comprend le refroidissement de la préforme en polyéthylène à une vitesse de refroidissement de 40°C par heure ou moins.

12. Procédé selon la revendication 1, dans lequel l'étape d'exposition au fluide supercritique est réalisée à une température située dans la plage de 50° à 250°C, de préférence de 80° à 130°C, pendant une durée d'au plus 4 heures.

13. Procédé selon la revendication 12, dans lequel l'étape d'exposition au fluide supercritique est réalisée à une pression située dans la plage de 3,45 à 27,6 MPa (500 à 4000 psi), de préférence de 6,89 à 20,68 MPa (1000 à 3000 psi).
